# EUROPEAN PATENT SPECIFICATION

(11) **EP 0 409 435 B1**
(45) Date of publication and mention of the grant of the patent: **26.10.1994**
(21) Application number: 90307166.0
(22) Date of filing: 29.06.1990
(51) Int. Cl.: C07D 403/14, C07D 413/14, C07D 417/14, A61K 31/495

(54) **Heteroaryl piperazine antipsychotic agents**
Heteroaryl Piperazine als antipsychotische Mittel
Hétéroaryl pipérazine comme agents antipsychotiques

(30) Priority: 07.07.1989 WO PCT/US89/02954
(43) Date of publication of application: 23.01.1991
(73) Proprietor: PFIZER INC., New York, N.Y. 10017 (US)
(72) Inventor: Howard, Harry R., Bristol, State of Connecticut (US)
(74) Representative: Moore, James William, Dr.

(56) References cited:
- EP-A- 0 281 309
- EP-A- 0 316 723
- EP-A- 0 318 933
- US-A- 4 831 031

## Description

The present invention is directed to novel heteroaryl piperazine compounds of the formula I, depicted below, which exhibit neuroleptic activity and are useful in the treatment of psychosis and anxiety.

Other compounds useful in treating psychotic disorders are known. For example, European Patent Application 0281309 states that compounds of the formula 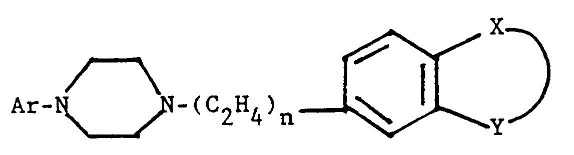
wherein Ar, n, X and Y are as defined in that application, are useful in treating psychotic disorders. The novel compounds of the present invention, however, exhibit substantially greater neuroleptic activity than such known compounds.

The present invention relates to compounds of the formula 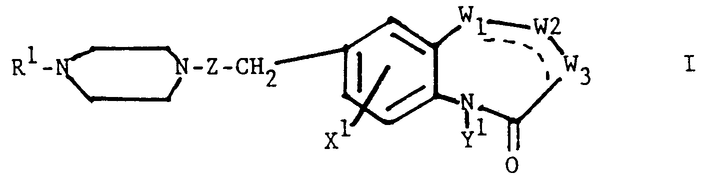
wherein W¹ is CR²R³, W² is CR⁴R⁵, W³ is CR⁶R⁷, and one of W¹, W² and W³ may be absent, and wherein the broken line extending from W¹ to W³ represents an optional bond between either W¹ and W² or W² and W³, in which case two of R², R³, R⁴, R⁵, R⁶ and R⁷ are absent; and wherein X¹ is hydrogen, halogen, (C₁-C₄) alkyl, (C₁-C₄)alkoxy, nitro, cyano, trifluoromethyl or pentafluoroethyl, or X¹ forms a heterocyclic ring with Y¹; Y¹ is hydrogen, (C₁-C₄) alkyl, phenyl or substituted phenyl, wherein said substituted phenyl is substituted with one or more substituents that are independently selected from the group consisting of halogen, (C₁-C₄) alkyl, nitro, cyano, (C₁-C₄) alkoxy, trifluoromethyl or pentafluoroethyl, or Y¹ forms a heterocyclic ring with X¹;
R¹ is 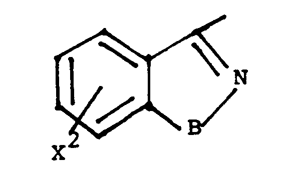
wherein B is selected from the group consisting of S, O and NY²; X² is hydrogen, halogen, (C₁-C₄) alkyl, (C₁-C₄) alkoxy, nitro, cyano, trifluoromethyl or pentafluoroethyl, or X² forms a heterocyclic ring with Y²; Y² is hydrogen, (C₁-C₄) alkyl, phenyl or substituted phenyl, wherein said substituted phenyl is substituted with one or more substituents that are independently selected from the group consisting of halogen, (C₁-C₄) alkyl, (C₁-C₄) alkoxy, nitro, cyano, trifluoromethyl or pentafluoroethyl, or Y² forms a heterocyclic ring with X²; R², R³ R⁴, R⁵, R⁶ and R⁷ are independently selected from the group consisting of hydrogen and lower alkyl, or any two of R², R³, R⁴, R⁵, R⁶ and R⁷ taken together with the carbon or carbons to which they are attached form a (C₃-C₇) saturated or unsaturated carbocyclic ring; and Z is (C₁-C₆) alkylene, branched (C₂-C₆)alkylene, (C₂-C₆)alkenylene or branched (C₃-C₆)alkenylene.

The present invention also relates to the pharmaceutically acceptable acid addition salts of the compounds of formula I. Such pharmaceutically acceptable acid addition salts include, but are not limited to the respective salts of acetic, malic, citric, fumaric, sulfuric, hydrochloric, hydrobromic, hydroiodic, sulfonic such as methanesulfonic and p-toluenesulfonic, and related acids.

Preferred compounds of the invention are:
6-(2-(4-(1,2-benzisothiazol-3-yl)piperazinyl)ethyl)-1,2,3,4-tetrahydro-2(1H)-quinolinone hydrochloride hemihydrate,
4(R,S)-methyl-6-(2-(4-(1,2-benzisothiazol-3-yl)piperazinyl)ethyl)-1,2,3,4-tetrahydro-2(1H)-quinolinone hydrochloride hydrate,
4S-methyl-6-(2-(4-(1,2-benzisothiazol-3-yl)piperazinyl)ethyl)-1,2,3,4-tetrahydro-2(1H)-quinolinone hydrochloride hydrate,
4R-methyl-6-(2-(4-(1,2-benzisothiazol-3-yl)piperazinyl)ethyl)-1,2,3,4-tetrahydro-2(1H)-quinolinone hydrochloride hydrate,
7-chloro-6-(2-(4-(1,2-benzisothiazol-3-yl)piperazinyl)ethyl)-1,2,3,4-tetrahydro-2(1H)-quinolinone quarterhydrate,
6-(3-(4-(1,2-benzisothiazol-3-yl)piperazinyl)propyl)-1,2,3,4-tetrahydro-4-methyl-2(1H)-quinolinone,
7-(2-(4-(1,2-benzisothiazol-3-yl)-piperazinyl)ethyl)-1,3,4,5-tetrahydro-2H-1-benzazepin-2-one,
1-ethyl-6-(2-(4-(1,2-benzisothiazol-3-yl)piperazinyl)ethyl)-1,2,3,4-tetrahydro-2(1H)-quinolinone, and
4,4-dimethyl-6-(2-(4-(1,2-benzisothiazol-3-yl)-piperazinyl)ethyl)-1,2,3,4-tetrahydro-2(1H)-quinoline.

Specific compounds of the invention are:
6-(2-(4-(1,2-benzisothiazol-3-yl)piperazinyl)ethyl)-1,2,3,4-tetrahydro-7-trifluoromethyl-2(1H)-quinolinone,
7-chloro-4-methyl-6-(2-(4-(1,2-benzisothiazol-3-yl)-piperazinyl)ethyl)-1,2,3,4-tetrahydro-2(1H)-quinolinone hydrochloride hydrate,
6-(2-(4-(1,2-benzisothiazol-3-yl)piperazinyl)ethyl)-1,2,3,4-tetrahydro-4-methyl-7-trifluoromethyl-2(1H)-quinolinone,
6-(2-(4-(1,2-benzisothiazol-3-yl)piperazinyl)ethyl)-1,2,3,4-tetrahydro-3,4-dimethyl-2(1H)-quinolinone,
6-(2-(4-(1,2-benzisothiazol-3-yl)piperazinyl)ethyl)-1,2,3,4-tetrahydro-5,7-dimethyl-2(1H)-quinolinone,
6′-(2-(4-(1,2-benzisothiazol-3-yl)piperazinyl)ethyl-1′,2′,3′,4′-tetrahydro-spiro[cyclopentane-1,4′-quinoline]-2′-one,
6′-(2-(4-(1,2-benzisothiazol-3-yl)piperazinyl)ethyl)-1′,2′,3′,4′-tetrahydro-spiro[cyclopropane-1,4′-quinoline]-2′-one,
6-(2-(4-(6-chloro-1,2-benzisothiazol-3-yl)piperazinyl)ethyl)-1,2,3,4-tetrahydro-4-methyl-2(1H)-quinolinone,
6-(2-(4-(6-fluoro-1,2-benzisothiazol-3-yl)piperazinyl)ethyl)-1,2,3,4-tetrahydro-4-methyl-2(1H)-quinolinone,
6-(2-(4-(5-fluoro-1,2-benzisothiazol-3-yl)piperazinyl)ethyl)-1,2,3,4-tetrahydro-4-methyl-2(1H)-quinolinone,
7-(2-(4-(1,2-benzisothiazol-3-yl)piperazinyl)ethyl)-1,3,4,5-tetrahydro-8-chloro-2H-1-benzazepin-2-one, and
7-(2-(4-(1,2-benzisothiazol-3-yl)piperazinyl)ethyl)-1,3,4,5-tetrahydro-5,5-dimethyl-2H-1-benzazepin-2-one.

The compounds of formula I may have optical centers and therefore may occur in different stereochemical configurations. The invention includes all stereoisomers of such compounds of formula I, including racemic mixtures thereof.

The invention also relates to pharmaceutical compositions for administration to a human which comprise a compound of the formula I or a pharmaceutically acceptable acid addition salt thereof, and a pharmaceutically acceptable carrier. Said pharmaceutically acceptable acid addition salts include but are not limited to those listed above.

Reaction scheme 1 below illustrates the preparation of compounds of formula I. Reaction scheme 2 below illustrates two methods of preparing compounds of the formula II, the starting material depicted in scheme 1. 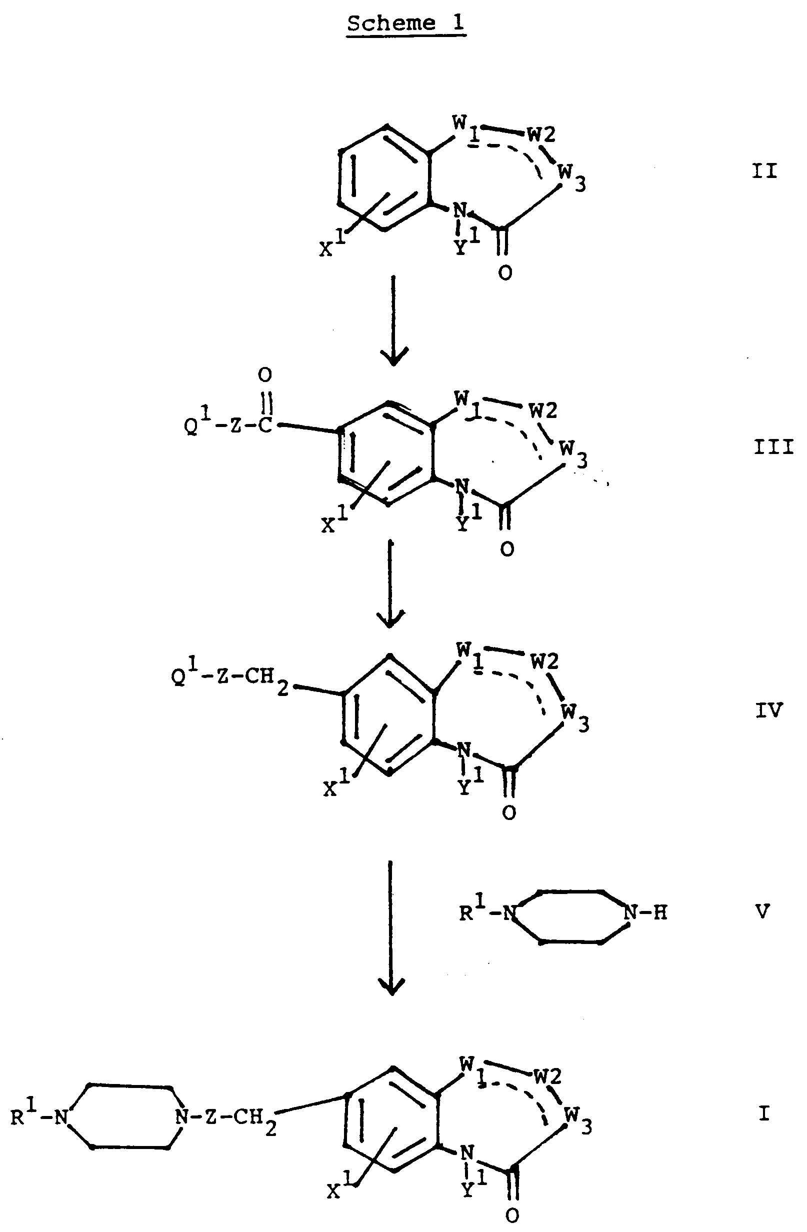 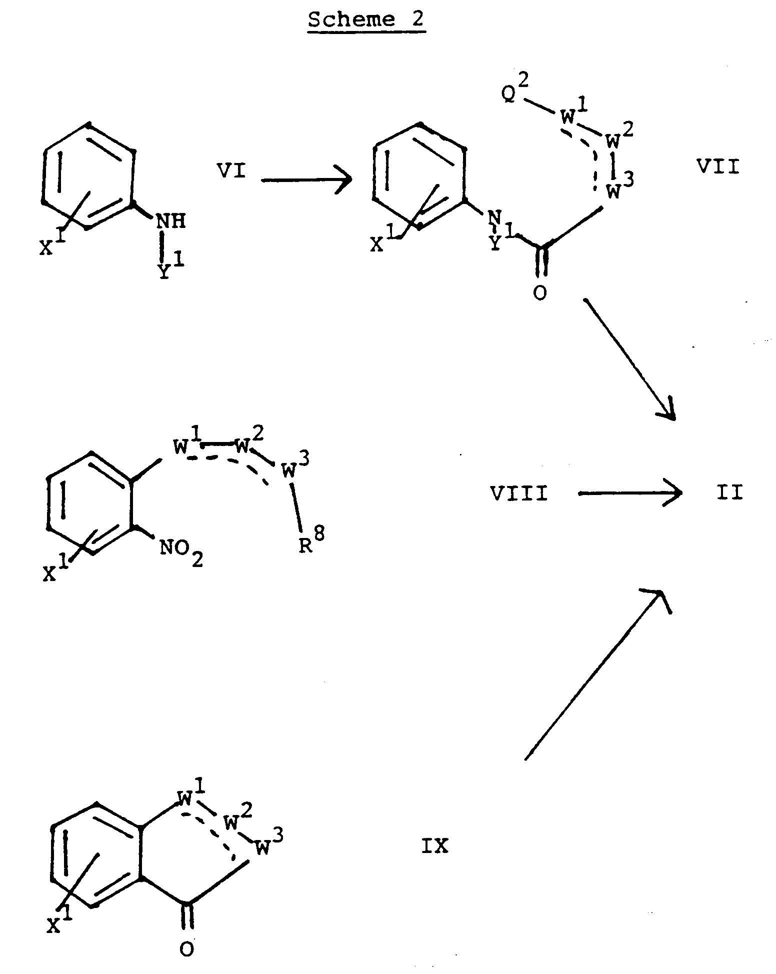
Compounds of the formula I, wherein R¹, W¹, W², W³, X¹, Y¹ and Z are as defined above, may be prepared by reacting piperazines of formula V, wherein R¹ is as defined above, with compounds of formula IV, wherein W¹, W², W³, X¹, Y¹ and Z are as defined above and Q¹ is a halogen (e.g., F, Br, Cl, I) or other suitable leaving group (e.g., CH₃SO₃, p-toluenesulfonyloxy). The reaction is generally performed in a polar solvent such as a lower alcohol, dimethylformamide, dimethylacetamide, acetonitrile, or methyl isobutyl ketone, and in the presence of a weak tertiary base such as triethylamine or an inorganic base such as sodium or potassium carbonate. A catalytic amount of sodium or potassium iodide can be employed to further the degree of completion. The reaction may be conducted at a temperature within the range of about 0°C to about 250°C, and preferably it is conducted at the reflux temperature (boiling point) of the chosen solvent.

The piperazine derivatives of the formula V may be prepared by methods known in the art and, in particular, as described by Lowe et al. in European Patent Application 0281309, in which an aryl or heteroaryl halide of the formula R-Hal (wherein Hal is F, Cl, Br, I) is reacted in an inert solvent (e.g., diglyme) at a temperature from about room temperature to about the reflux temperature of the selected solvent for about one half to about 48 hours and preferably for about 16-24 hours.

Compounds of the formula IV may be prepared from compounds of the formula III, wherein Q¹, W¹, W², W³, X¹, Y¹ and Z are as defined above, by methods available to those practicing in the art and analogous to those described in European Patent Application 0281309. Thus, compounds of the formula IV may be obtained by reducing a compound of the formula III with a reducing agent such as triethylsilane in trifluoroacetic acid.

Compounds of the formula III may be obtained by reacting a compound of the formula II, wherein W¹, W², W³, X¹ and Y¹ are as defined above, with a haloalkanoic acid or a haloalkanoyl halide, wherein the halogen is selected from the group consisting of F, Cl, Br and I, employing, for example, Friedel-Crafts conditions (e.g., aluminum trichloride in carbon disulfide or methylene dichloride under an inert atmosphere) or via acylation in a medium such as polyphoshoric acid at a temperature from about room temperature to about 100°C.

The preparation of the compounds of the formula II used in the above process can be accomplished by several methods, as described in the literature and outlined in scheme 2. Referring to scheme 2, an aryl amine of the formula VI, wherein X¹ and Y¹ are as defined above, can be converted, using methods known in the art, to an arylamide of the formula VII, wherein W¹, W², W³, X¹ and Y¹ are as defined above and Q² is defined as Q¹ above, which may then be cyclized to produce a compound of the formula II.

Compounds of the formula II, wherein a carbon-carbon double bond exists between either W¹ and W² or W² and W³ may be reduced using known methods such as catalytic hydrogenation or reduction with magnesium metal in methanol to produce compounds of the formula II, wherein the corresponding bond between either W¹ and W² or W² and W³ is a carbon-carbon single bond.

Compounds of the formula II, wherein W¹, W², W³ and X¹ are as defined above and Y¹ is hydrogen, can alternatively be prepared by other known methods, as illustrated in scheme 2. For example, they can be prepared by concomitantly reducing and cyclizing a compound of the formula VIII, wherein W¹, W², W³, and X¹ are as defined above and R⁸ is a nitrile, a carboxylic acid or a carboxylate such as a methyl or ethyl ester. They may also be prepared from compounds of the formula IX, wherein W¹, W², W³, and X¹ are as defined above, using such known methods as Schmidt or Beckmann rearrangements.

The pharmaceutically acceptable acid addition salts of the compounds of formula I are prepared in a conventional manner by treating a solution or suspension of the free base, i.e. a compound of formula I, with about one chemical equivalent of a pharmaceutically acceptable acid. Conventional concentration and recrystallization techniques are employed in isolating the salts.

The neuroleptic activity of the present compounds may be demonstrated by methods based on standard procedures. In one method, adult male Sprague-Dawley rats are pretreated with appropriate doses of the test compound by subcutaneous injection. One half hour later all rats are injected intraperitoneally with 1 mg/kg apomorphine hydrochloride dissolved in an 0.1% ascorbate solution. The rats are rated behaviorally according to the following scale at 5, 15, 25, 35 and 45 minutes after the apomorphine injection: 0 = alert but not moving, 1 = moving around the cage, 2 = discontinuous sniffing behavior, 3 = continuous sniffing with discontinuous oral movements, and 4 = continuous licking and chewing movements.

The neuroleptic activity of the compounds of this invention makes them useful for treating psychotic disorders in human subjects. For example, these compounds are useful for treating psychotic disorders of the schizophrenic types and in particular the compounds are useful for removing or ameliorating such symptoms as anxiety, agitation, excessive aggression, tension and social or emotional withdrawal in psychotic patients.

A neuroleptic compound of the formula I or a pharmaceutically-acceptable salt thereof can be administered to a human subject either alone or preferably in combination with pharmaceutically-acceptable carriers or diluents in a pharmaceutical composition according to standard pharmaceutical practice. A compound can be administered orally or parenterally. Parenteral administration includes especially intravenous and intramuscular administration. Additionally, in a pharmaceutical composition comprising a compound of formula I or a pharmaceutically-acceptable salt thereof, the weight ratio of active ingredient to carrier will normally be in the range from about 1:6 to about 2:1 and preferably from about 1:4 to about 1:1. However, in any given case, the ratio chosen will depend on such factors as the solubility of the active component, the dosage contemplated and the precise route of administration.

For oral use of a neuroleptic agent of this invention, the compound can be administered, for example, in the form of tablets or capsules or as an aqueous solution or suspension. In the case of tablets for oral use, carriers which can be used include lactose and corn starch, and lubricating agents such as magnesium stearate can be added. For oral administration in capsule form, useful diluents are lactose and dried corn starch. When aqueous suspensions are required for oral use, the active ingredient can be combined with emulsifying and suspending agents. If desired, certain sweetening and or flavoring agents can be added. For intramuscular and intravenous use, sterile solutions of the active ingredient can be prepared and the pH of the solutions should be suitably adjusted and buffered. For intravenous use the total concentration of solutes should be controlled to render the preparation isotonic.

When a neuroleptic agent of this invention is to be used in a human subject to treat a psychotic disorder, the daily dosage will normally be determined by the prescribing physician. Moreover, the dosage will vary according to the age, weight and response of the individual patient as well as the severity of the patient's symptoms. However, in most instances an effective amount for treating a psychotic disorder will be a daily dosage in the range from about 3 mg to about 600 mg and preferably from about 30 mg to about 60 mg in single or divided doses, orally or parenterally. In some instances, it may be necessary to use dosages outside these limits.

The present invention is illustrated by the following examples, but is not limited to the details thereof.

The title compounds of examples 37 through 55 are compounds of this invention. The title compounds of examples 1 through 18 are compounds of the formula III above. The title compounds of examples 19 through 36 are compounds of the formula IV above. The title compounds of examples 56 through 69 are compounds of the formula II above.

### Example 1

### 6-(Chloroacetyl)-1,2,3,4-tetrahydro-2(1H)-quinolinone (C₁₁H₁₀ClNO₂)

Under nitrogen a mixture of 5.2 ml (0.065 mol) chloroacetyl chloride and 41.4 g (0.31 mol) aluminum trichloride in 200 ml carbon disulfide was stirred while adding 7.36 g (0.05 mol) 1,2,3,4-tetrahydro-2(1H)-quinolinone over a 5 minute period. After a further 15 minute period, the mixture was refluxed for 2 hours, treated with another 20 ml (0.25 mol) chloroacetyl chloride and refluxed another 3 hours. The reaction mixture was cooled to 25°C, the carbon disulfide was decanted and the viscous brown oil was poured cautiously over 500 g ice/water. After stirring for 30 minutes, the precipitated solids were filtered, washed well with water and air dried to give 10.75 g of an off-white solid, (96%), m.p. 215-218°C (dec.); MS (%) 223(9), 174(100), NMR (δ, DMSO-d₆) 2.0-2.35 (m,2H), 2.45-2.8 (m,2H), 4.7 (s,2H), 6.6 (d,1H), 7.4-7.6 (m,2H), 10.0 (br s,1H).

The 6-chloroacetyl-1,2,3,4-tetrahydro-2(1H)-quinolinones of Examples 2-16 were prepared by a procedure similar to that of Example 1.

### Example 2

### 1-Ethyl-6-chloroacetyl-1,2,3,4-tetrahydro-2(1H)-quinolinone

C₁₃H₁₄ClNO₂, 98%, m.p. 158-161°C.

### Example 3

### 4(R,S)-Methyl-6-chloroacetyl-1,2,3,4-tetrahydro-2(1H)-quinolinone

C₁₂H₁₂ClNO₂, 48%, m.p. 183-184°C.

### Example 4

### 4R-Methyl-6-chloroacetyl-1,2,3,4-tetrahydro-2(1H)-quinolinone

C₁₂H₁₂ClNO₂, 87%, m.p. 187-190°C, [α]²⁵D + 2.1° (C=1, acetone).

### Example 5

### 4S-Methyl-6-chloroacetyl-1,2,3,4-tetrahydro-2(1H)-quinolinone

C₁₂H₁₂ClNO₂, 92%, m.p. 187-190°C, [α]²⁵D - 5.9° (C=1, acetone).

### Example 6

### 3-Methyl-6-chloroacetyl-1,2,3,4-tetrahydro-2(1H)-quinolinone

C₁₂H₁₂ClNO₂, 96%, m.p. 216-221°C.

### Example 7

### 7-Methyl-6-chloroacetyl-1,2,3,4-tetrahydro-2(1H)-quinolinone

C₁₂H₁₂ClNO₂, 91%, m.p. 196-199°C.

### Example 8

### 3,3-Dimethyl-6-chloroacetyl-1,2,3,4-tetrahydro-2(1H)-quinolinone

C₁₃H₁₄ClNO₂, 97%, m.p. 204-206°C.

### Example 9

### 4,4-Dimethyl-6-chloroacetyl-1,2,3,4-tetrahydro-2(1H)-quinolinone

C₁₃H₁₄ClNO₂, 98%, m.p. 175-177°C.

### Example 10

### 4,7-Dimethyl-6-chloroacetyl-1,2,3,4-tetrahydro-2(1H)-quinolinone

C₁₃H₁₄ClNO₂, 92%, m.p. 184-186°C.

### Example 11

### 1,4-Dimethyl-6-chloroacetyl-1,2,3,4-tetrahydro-2(1H)-quinolinone

C₁₃H₁₄ClNO₂, 88%, m.p. 122-124°C.

### Example 12

### 1,3,3-Trimethyl-6-chloroacetyl-1,2,3,4-tetrahydro-2-(1H)-quinolinone

C₄H₁₆ClNO₂, 94%, oil.

### Example 13

### 4,4,7-Trimethyl-6-chloroacetyl-1,2,3,4-tetrahydro-2-(1H)-quinolinone

C₁₄H₁₆ClNO₂, 95%, m.p. 176-179°C.

### Example 14

### 7-Chloro-6-chloroacetyl-1,2,3,4-tetrahydro-2(1H)-quinolinone

C₁₁H₉Cl₂NO₂, 58%, m.p. 208-211°C.

### Example 15

### 7-Chloro-4,4-dimethyl-6-chloroacetyl-1,2,3,4-tetrahydro-2(1H)-quinolinone

C₁₃H₁₃Cl₂NO₂, 57%, m.p. 153-156°C.

### Example 16

### 7-Chloro-1-ethyl-6-chloroacetyl-1,2,3,4-tetrahydro-2-(1H)-quinolinone

C₁₃H₁₄Cl₂NO₂, 60%, m.p. 109-111°C.

### Example 17

### 6-(3-Chloropropionyl)-1,2,3,4-tetrahydro-4-methyl2(1H)-quinolinone

The title compound was prepared by a procedure similar to that of Example 1, but replacing chloroacetyl chloride with 3-chloropropionyl chloride.

C₁₃H₁₄ClNO₂, 96%,m.p. 134-136°C.

### Example 18

### 7-Chloroacetyl-1,2,4,5-tetrahydro-2H-1-benzazepin-2-one

The title compound was prepared from 1,3,4,5-tetrahydro-2H-1-benzazepin-2-one by a procedure similar to that of Example 1.

### Example 19

### 6-(2-Chloroethyl)-1,2,3,4-tetrahydro-2(1H)-quinolinone

Under nitrogen, a mixture of 6.71 g of the title compound of Example 1 and 23 ml (0.30 mol) trifluoroacetic acid was treated dropwise with 11.0 ml (0.069 mol) triethylsilane. After 72 hours at 25°C the dark brown solution was poured slowly over 200 ml ice, stirred for 30 minutes and filtered. The solids were washed well with water and dried to give a tan colored product, 5.42 g (86%), m.p. 148-153°C (dec.); MS(%) 211(10), 209(34), 160(100), 132(45); NMR (δ, DMSO-d₆) 2.0-2.3 (m,2H), 2.4-2.75 (m,4H), 3.4 (t,2H), 6.40 (d, 1H), 6.6-6.8 (m,2H), 9.7 (br s,1H).

By a process similar to that of example 19, the following "R" substituted 6-(2-chloroethyl)-1,2,3,4-tetrahydro-2(1H)-quinolinones of examples 20 through 34 were prepared.

| Example | "R" | Molecular Formula | m.p. °C | Yield % | MS (%)/[α] ²⁵D |
|---|---|---|---|---|---|
| 20 | 1-ethyl | C₁₃H₁₆ClNO | 55-58 | 94 | |
| 21 | 4(R,S)-methyl | C₁₂H₁₄ClNO | 176-178 | 87 | |
| 22 | 4R-methyl | C₁₂H₁₄ClNO | 176-178 | 84 | /+ 8.9° c=1, acetone |
| 23 | 4S-methyl | C₁₂H₁₄ClNO | 176-178 | 88 | /- 8.5° c=1, acetone |
| 24 | 3-methyl | C₁₂H₁₄ClNO | 136-140 | 70 | |
| 25 | 7-methyl | C₁₂H₁₄ClNO | 233-234 | 57 | |
| 26 | 3,3-dimethyl | C₁₃H₁₆ClNO | 136-138 | 78 | |
| 27 | 4,4-dimethyl | C₁₃H₁₆ClNO | 175-178 | 61 | |
| 28 | 4,7-dimethyl | C₁₃H₁₆ClNO | 178-180 | 69 | |
| 29 | 1,4-dimethyl | C₁₃H₁₆ClNO | 77-79 | 83 | |
| 30 | 1,3,3-trimethyl | C₁₄H₁₈ClNO | Oil | 84 | 251(77), 202(100)/ |
| 31 | 4,4,7-trimethyl | C₁₄H₁₈ClNO | 203-207 | 86 | |
| 32 | 7-chloro | C₁₁H₁₁Cl₂NO | 238-240 | 63 | |
| 33 | 7-chloro-4,4-dimethyl | C₁₃H₁₅Cl₂NO | 194-196 | 85 | |
| 34 | 7-chloro-1-ethyl | C₁₃H₁₅Cl₂NO | 94-97 | 52 | |

### Example 35

### 6-(3-chloropropyl)-1,2,3,4-tetrahydro-4-methyl-2(1H)-quinolinone

Using a procedure similar to that of Example 19, the title compound was prepared by reducing 6-(3-chloropropionyl)-1,2,3,4-tetrahydro-4-methyl-2(1H)-quinolinone, C₁₃H₁₆ClNO, 70%, m.p.92-94°C.

### Example 36

### 7-(2-Chloroethyl)-1,3,4,5-tetrahydro-2H-1-benzazepin-2-one

Using a procedure similar to that of Example 19, the title compound was prepared by reducing 7-chloroacetyl-1,3,4,5-tetrahydro-2H-1-benzazepin-2-one, C₁₂H₁₄ClNO, oil, 83%.

### Example 37

### 6-(2-(4-(1,2-benzisothiazol-3-yl)piperazinyl)ethyl-1,2,3,4-tetrahydro-2(1H)-quinolinone hydrochloride hemihydrate

Under nitrogen a mixture of 1.097 g (5.0 mmol) 1-(1,2-benzisothiazol-3-yl)piperazine, 1.05 g (5.0 mmol) 6-(2-chloroethyl)-1,2,3,4-tetrahydro-2(1H)-quinolinone, 1.06 g (10.0 mmol) sodium carbonate, 0.083 g (0.5 mmol) potassium iodide and 35 ml methyl isobutyl ketone (MIBK) was heated at 90°C for 18 hours. After cooling to 25°C, the mixture was filtered, the solids were washed with another 100 ml MIBK and the combined filtrates were concentrated in vacuo to an orange solid. After chromatography on silica gel (230-400 mesh, 45x160 mm), eluting with ethyl acetate, the product fractions were combined and concentrated in vacuo, diluted in 15 ml methylene dichloride and treated with hydrogen chloride saturated ethyl ether to give a pale yellow solid, 0.280 g (13%), m.p. 285-288°C; MS(%) 392(1), 232(100), 177, 160; Anal. for C₂₂H₂₄N₄OS · HCl·1/2H₂O: C 60.33, H 5.98, N 12.79. Found: C 59.98, H 5.84, N 12.66.

Using a procedure similar to that of Example 37, the 6-(2-(4-(1,2-benzisothiazol-3-yl)piperazinyl)ethyl)-1,-2,3,4-tetrahydro-2(1H)-quinolinones of examples 38-52 were prepared.

### Example 38

### 1-Ethyl-6-(2-(4-(1,2-benzisothiazol-3-yl)piperazinyl)-ethyl)-1,2,3,4 tetrahydro-2(1H)-quinolinone

70%, m.p. 94°C, MS(%): 420(1), 232(100).

### Example 39

### 4(R,S)-Methyl-6-(2-(4-(1,2-benzisothiazol-3-yl)-piperazinyl)ethyl)-1,2,3,4 tetrahydro-2(1H)-quinolinone

37%, m.p. 241-243°C, Anal. for C₂₃H₂₆N₄OS·HCl·H₂O: C, 59.92, H, 6.34, N, 12.15. Found: C, 59.87, H, 6.39, N, 11.88.

### Example 40

### 4R-Methyl-6-(2-(4-(1,2-benzisothiazol-3-yl)-piperazinyl)ethyl)-1,2,3,4 tetrahydro-2(1H)-quinolinone

77%, m.p. 246°C; Anal. for C₂₃H₂₆N₄OS·HCl·H₂O: C, 59.92, H, 6.34, N, 12.15. Found: C, 60.24, H, 6.27, N, 11.88.

### Example 41

### 4S-Methyl-6-(2-(4-(1,2-benzisothiazol-3-yl)-piperazinyl)ethyl)-1,2,3,4 tetrahydro-2(1H)-quinolinone

78%, m.p. 246-248°C; Anal. for C₂₃H₂₆N₄OS·HCl·H₂O: C, 59.92, H, 6.34, N, 12.15. Found: C, 59.66, H, 6.45, N, 11.78.

### Example 42

### 3-Methyl-6-(2-(4-(1,2-benzisothiazol-3-yl)-piperazinyl)ethyl)-1,2,3,4 tetrahydro-2(1H)-quinolinone

59%, m.p. 190-192°C; Anal. for C₂₃H₂₆N₄OS: C, 67.95, H, 6.45, N, 13.78. Found: C, 67.73, H, 6.47, N, 13.33.

### Example 43

### 7-Methyl-6-(2-(4-(1,2-benzisothiazol-3-yl)-piperazinyl)ethyl)-1,2,3,4 tetrahydro-2(1H)-quinolinone

24%, m.p. 200°C (dec.); Anal. for C₂₃H₂₆N₄OS·1/2H₂O: C, 66.48, H, 6.55, N,13.48. Found: C, 66.88, H, 6.33, N, 13.45.

### Example 44

### 3,3-Dimethyl-6-(2-(4-(1,2-benzisothiazol-3-yl)-piperazinyl)ethyl)-1,2,3,4 tetrahydro-2(1H)-quinolinone

59%, m.p. 195-198°C; Anal. for C₂₄H₂₈N₄OS·1/2H₂O: C, 67.10, H, 6.80, N, 13.04. Found: C, 66.92, H, 6.75, N, 12.81.

### Example 45

### 4,4-Dimethyl-6-(2-(4-(1,2-benzisothiazol-3-yl)-piperazinyl)ethyl)-1,2,3,4 tetrahydro-2(1H)-quinolinone

78%, m.p. 264°C (dec.); Anal. for C₂₄H₂₈N₄OS·HCl·H₂O: C, 60.68, H, 6.58, N, 11.79. Found: C, 60.33, H, 6.35, N, 11.47;

### Example 46

### 4,7-Dimethyl-6-(2-(4-(1,2-benzisothiazol-3-yl)-piperazinyl)ethyl)-1,2,3,4 tetrahydro-2(1H)-quinolinone

38%, m.p. 189-191°C; Anal. for C₂₄H₂₈N₄OS·1/4H₂O: C, 67.81, H, 6.76, N, 13.18. Found: C, 67.98, H, 6.78, N, 13.01;

### Example 47

### 1,4-Dimethyl-6-(2-(4-(1,2-benzisothiazol-3-yl)-piperazinyl)ethyl)-1,2,3,4 tetrahydro-2(1H)-quinolinone

47%, m.p. 251-252°C (dec.); Anal. for C₂₄H₂₈N₄OS·HCl: C, 63.01, H, 6.40, N, 12.26. Found: C, 62.65, H, 6.24, N, 11.87.

### Example 48

### 1,3,3-Trimethyl-6-(2-(4-(1,2-benzisothiazol-3-yl)-piperazinyl)ethyl)-1,2,3,4 tetrahydro-2(1H)-quinolinone

44%, m.p. 259-263°C; Anal. for C₂₅H₃₀N₄OS·HCl·1/3H₂O: C, 62.94, H, 6.69, N, 11.74. Found: C, 62.95, H, 6.51, N, 11.60.

### Example 49

### 4,4,7-Trimethyl-6-(2-(4-(1,2-benzisothiazol-3-yl)piperazinyl)ethyl)-1,2,3,4 tetrahydro-2(1H)-quinolinone

56%, m.p. 257°C (dec.); Anal. for C₂₅H₃₀N₄OS·HCl: C, 61.39, H, 6.80, N, 11.46. Found: C, 61.59, H, 6.61, N, 11.10.

### Example 50

### 7-Chloro-6-(2-(4-(1,2-benzisothiazol-3-yl)-piperazinyl)ethyl)-1,2,3,4 tetrahydro-2(1H)-quinolinone

52%, m.p. 212-215°C; Anal. for C₂₂H₂₃ClN₄OS·1/4H₂O: C, 61.24, H, 5.49, N, 12.99. Found: C, 61.30, H, 5.43, N, 12.72.

### Example 51

### 7-Chloro-4,4-dimethyl-6-(2-(4-(1,2-benzisothiazol-3-yl)piperazinyl)ethyl)-1,2,3,4 tetrahydro-2(1H)-quinolinone

56%, m.p. 290-292°C; Anal. for C₂₄H₂₇ClN₄OS·HCl: C, 58.65, H, 5.74, N, 11.40. Found: C, 58.29, H, 5.69, N, 11.25.

### Example 52

### 7-Chloro-1-ethyl-6-(2-(4-(1,2-benzisothiazol-3-yl)-piperazinyl)ethyl)-1,2,3,4 tetrahydro-2(1H)-quinolinone

61%, m.p. 281°C (dec.); Anal. for C₂₄H₂₇ClN₄OS·HCl·1/4H₂O: C, 58.12, H, 5.79, N, 11.30. Found: C, 58.06, H, 5.63, N, 10.97.

### Example 53

### 6-(3-(4-(1,2-Benzisothiazol-3-yl)-piperazinyl)propyl)-1,2,3,4-tetrahydro-4-methyl-2(1H)-quinolinone

The title compound was prepared in manner similar to that of Example 37. 79%, m.p. 156-157°C, Anal. for C₂₄H₂₈N₄OS: C, 68.54, H, 6.71, N, 13.32. Found: C, 68.36, H, 6.64, N, 13.30.

### Example 54

### 7-(2-(4-(1,2-Benzisothiazol-3yl)-piperazinyl)ethyl)-1,-3,4,5-tetrahydro-2H-1-benzazepin-2-one

The title compound was prepared by a procedure similar to that of Example 37. 23%, m.p. 173-174.5°C.

### Example 55

### 7-(2-(4-(1,2-Benzisothiazol-3-yl)piperazinyl)ethyl)-1,-3,4,5-tetrahydro-1-methyl-2H-1-benzazepin-2-one

Under nitrogen, sodium hydride (20 mg, 0.5 mmol, 60% oil dispersed) was washed free of oil with pentane and layered with 6ml dimethylformamide. In one portion, 155 mg (0.38 mmol) 7-(2-(4-(1,2-benzisothiazol-3-yl)piperazinyl)ethyl)-1,3,4,5-tetrahydro-2H-1-benzazepin-2-one (the title compound of Example 54) was added and stirring was continued for 0.5 hours at 25°C. To the solution was then added methyl iodide (162 mg, 1.14 mmol) and stirring was continued overnight. After pouring over 60 ml ice/water, the product was extracted with 40 ml ethyl acetate which was washed with water (2x50 ml), dried with sodium sulfate and concentrated to an oil, 54 mg. Chromatography (32-63 micron silica gel) eluting with 2% methanol in methylene dichloride provided the pure free base, 46 mg (29%).

### Example 56

### 1,2,3,4-Tetrahydro-4(R,S)-methyl-2(1H)-quinolinone

The title compound was prepared according to the method of R. Brettle and S.M. Shibib, J. Chem. Soc., Part I, 2912-2919, (1981). Thus, 2-hydroxy-4-methylquinoline (Aldrich) in methanol was reduced with magnesium metal, white solid, 47%, m.p. 98-101°C (lit. m.p. 97-98°C).

### Example 57

### 4,7-Dimethyl-1,2,3,4-tetrahydro-4(R,S)-methyl-2(1H)-quinoline

The title compound was prepared in a manner similar to that of Example 56, m.p. 117-118.5°C, 42%, MS(%): 175 (60, M⁺), 160(100).

According to the procedure of A. Kraska et al., European Patent Application 0130795, the "R" substituted 1,2,3,4-tetrahydro-2(1H)-quinolinones of Examples 58-60 were prepared:

| Example | "R" | m.p. (°C) | Yield (%) |
|---|---|---|---|
| 58 | 4,4-dimethyl | 97-101 | 85 |
| 59 | 4,4,7-trimethyl | 115-118 | 34 |
| 60 | 7-chloro-4,4-dimethyl | 168-171 | 50 |

According to the method of D.W. Robertson, et al (J. Med. Chem., 29(10) 1832-1840 (1986), 1,2,3,4-tetrahydro-2(1H)-quinolinone (dihydrocarbostyril) was converted to the "R" substituted 1,2,3,4-tetrahydro-2(1H)-quinolinones of Examples 61-62.

| Example | "R" | m.p. (°C) | Yield (%) | NMR |
|---|---|---|---|---|
| 61 | 3,3-dimethyl | 154-156 | 49 | |
| 62 | 1,3,3-trimethyl | Oil | 44 | ¹H-NMR(300 MHz, δ , CDCl₃): 1.15 (S,6H), 2.7 (s, 2H), 3.35 (s,3H), 6.9 (d,1H), 7.0 (t,1H), 7.1 (d,1H), 7.2 (t,1H) |

### Example 63

### 7-Chloro-1,2,3,4-tetrahydro-2(1H)-quinolinone

To a solution of 6.58 g (28.9 mmole) of 4-chloro-2-nitrocinnamic acid (prepared according to the method of G. R. Pettit and A. B. Neill, Can. J. Chem., 42, 1764-1768 (1964)), in 150 ml ethanol and 4 ml acetic acid was added 1 level teaspoon of Raney nickel and the mixture was hydrogenated at 40-50 psi/25°C for 3 hours. The mixture was filtered through a pad of diatomaceous earth and the filtrate was concentrated to a crude solid. This solid was triturated with ethyl acetate and filtered to give the product as a white solid, 4.06 g (77%, m.p. 184-186°C, MS(%): 183(33, M⁺²), 181(100, M⁺).

### Example 64

### 7-Methyl-1,2,3,4-tetrahydro-2(1H)-quinolinone

The title compound was prepaed according to the method of T. Kametani, H. Nemoto and S. Takano, Chem. Pharm. Bull., 16(2), 367-370 (1968) m.p. 158-160°C (lit. m.p. 160-161°C).

### Example 65

### 1-Ethyl-1,2,3,4-tetrahydro-2(1H)-quinolinone

The title compound was prepared by stirring 11.3 mmol of 1,2,3,4-tetrahydro-2(1H)-quinolinone in 85 ml dry dimethylformamide with 12.1 mmol potassium tert-butoxide for two hours at 25°C, then adding 12.1 mmol ethyl iodide and refluxing for 20 hours. The product was isolated by pouring the reaction mixture over ice/water (about 200 ml), stirring until the ice melted, extracting with diethyl ether and drying the organic extracts over magnesium sulfate. The residue obtained on concentrating the organics was chromatographed (230-400 mesh silica gel, 75% hexane: 25% ethyl acetate). The product obtained was an oil, 56%.

### Example 66

### 7-Chloro-1-ethyl-1,2,3,4-tetrahydro-2(1H)-quinolinone

The title compound was prepared in a manner similar to Example 65, but using 7-chloro-1,2,3,4-tetrahydro-2(1H)-quinolinone (the title compound of Example 63) instead of 1,2,3,4-tetrahydro-2(1H)-quinolinone. The product obtained was an oil, 28%.

### Example 67

### 1,4-Dimethyl-1,2,3,4-tetrahydro-2(1H)-quinolinone

The title compound was prepared from 1,2,3,4-tetrahydro-4(R,S)-methyl-2(1H)-quinolinone in a manner similar to that of Example 65, but using methyl iodide instead of ethyl iodide and using potassium tert-butoxide as the base. The product so obtained was an oil (52%, MS(%): 175(100, M⁺), 160(95), 132(87)).

### Example 68

### 4(S)-Methyl-1,2,3,4-tetrahydro-2(1H)-quinolinone

3-phenylbutyric acid (Aldrich Chem Co.) was resolved according to the method of A. Weidler and G. Bergson (Acta Chem. Scand., 1964, 18(6), 1484-1486) into the corresponding enantiomers:
3S-(+)-phenylbutyric acid (oil, [α]²⁵D +50.7° (c=1, benzene)) and 3R-(-)-phenylbutyric acid (oil, [α]²⁵D -49.4° (c=1, benzene)).

Polyphosphoric acid (132 g, Aldrich) was preheated to 100°C in an open beaker and 3(S)-(+)-phenylbutyric acid (13.2 g, 80.4 mmol) was added. After 3 hours of mechanical stirring at 100°C the mixture was cooled to approximately 50°C and poured over 600 ml ice/water. The resulting oil was extracted with ethyl acetate, dried over magnesium sulfate and concentrated to a dark orange oil, 11.2 g. Distillation at 94°C/1.3 mm mercury gave pure 3(S)-methyl-1-indanone (6.77 g, 58%, [α]²⁵D +15.9° (c=1, acetone)), as reported by H.J. Hansen, Helv. Chim. Acta, 1979, 62(4), 1120-1128. In the same way, cyclization of 3R-(-)-phenylbutyric acid (14.9 g) in 149 g polyphosphoric acid gave, after distillation, pure 3R-methyl-1-indanone (9.52 g, 72%, [α]²⁵D -16.7° (c=1, acetone)).

According to a procedure in J. Org. Chem., 1958, 23, 1330, 3(S)-(+)-methyl-1-indanone (6.7 g, 45.8 mmol) in 100 g polyphosphoric acid was treated with sodium azide (3.12 g, 48.1 mmol) in small portions over a 30 minute period, then heated to 50°C with continued mechanical stirring overnight. The yellow viscous reaction mixture was cooled to 25°C, poured over 600 ml ice/water and made alkaline with 2 normal sodium hydroxide (to pH 8-9). This was extracted with methylene dichloride; the combined organic extracts were washed sequentially with saturated aqueous solutions of sodium bicarbonate and sodium chloride, and finally dried with magnesium sulfate and concentrated to a residue, 6.7 g. Chromatography (230-400 silica gel, 60% hexane: 40% ethyl acetate) provided pure 4(S)-methyl-1,2,3,4-tetrahydro-2(1H)-quinolinone as a white solid, 3.2 g (43%, m.p. 92-94°C, [α]²⁵D -38.8° (c=1, acetone).

### Example 69

### 4(R)-Methyl-1,2,3,4-tetrahydro-2(1H)-quinolinone

The title compound was prepared in a manner similar to that of Example 68, m.p. 93-96°C, [α]²⁵D + 36.9°, (c=1, acetone).

## Claims (Claims for the following Contracting State(s): AT, BE, CH, DE, DK, FR, GB, IT, LI, LU, NL, SE)

1. A compound having the formula 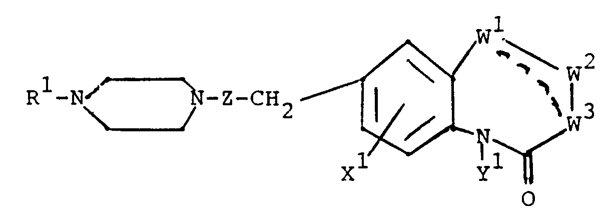 wherein W¹ is CR²R³, W² is CR⁴R⁵, W³ is CR⁶R⁷, and one of W¹, W² and W³ may be absent, and wherein the broken line extending from W¹ to W³ represents an optional bond between either W¹ and W² or W² and W³, in which case two of R², R³, R⁴, R⁵, R⁶ and R⁷ are absent; and wherein X¹ is hydrogen, halogen, (C₁-C₄)alkyl, (C₁-C₄)alkoxy, nitro, cyano, trifluoromethyl or pentafluoroethyl, or X¹ forms a heterocyclic ring with Y¹; Y¹ is hydrogen, (C₁-C₄) alkyl, phenyl or substituted phenyl, wherein said substituted phenyl is substituted with one or more substituents that are independently selected from halogen, (C₁-C₄) alkyl, (C₁-C₄) alkoxy, nitro, cyano, trifluoromethyl or pentafluoroethyl, or Y¹ forms a heterocyclic ring with X¹;
R¹ is 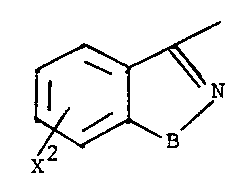 wherein B is selected from S, O and NY²; X² is hydrogen, halogen, (C₁-C₄) alkyl, (C₁-C₄) alkoxy, nitro, cyano, trifluoromethyl or pentafluoroethyl, or X² forms a heterocyclic ring with Y²; Y² is hydrogen (C₁-C₄) alkyl, phenyl or substituted phenyl, wherein said substituted phenyl is substituted with one or more substituents that are independently selected from halogen, (C₁-C₄) alkyl, (C₁-C₄) alkoxy, nitro, cyano, trifluoromethyl or trifluoroethyl, or Y² forms a heterocyclic ring with X²; R², R³, R⁴, R⁵, R⁶ and R⁷ are independently selected from hydrogen and lower alkyl, or any two of R², R³, R⁴, R⁵, R⁶ and R⁷ taken together with the carbon or carbons to which they are attached form a (C₃-C₇) saturated or unsaturated carbocyclic ring; and Z is (C₁-C₆) alkylene, branched (C₂-C₆)alkylene,(C₂-C₆)alkenylene or branched (C₃-C₆)alkenylene,
and the pharmaceutically acceptable salts thereof.

2. A compound according to claim 1, with the proviso that when Y¹ is hydrogen and W³ is absent, then the heterocyclic ring carbons of W¹ and W² are connected by a carbon-carbon single bond.

3. A compound according to claim 1, wherein Y¹ is hydrogen, W³ is absent and the heterocyclic ring carbons of W¹ and W² are connected by a carbon-carbon double bond.

4. A compound according to claim 1, said compound being selected from:
6-(2-(4-(1,2-benzisothiazol-3-yl)piperazinyl)ethyl)-1,2,3,4-tetrahydro-2(1H)-quinolinone hydrochloride hemihydrate,
4(R,S)-methyl-6-(2-(4-(1,2-benzisothiazol-3-yl)-piperazinyl)ethyl)-1,2,3,4-tetrahydro-2(1H)-quinolinone hydrochloride hydrate,
4S-methyl-6-(2-(4-(1,2-benzisothiazol-3-yl)-piperazinyl)ethyl)-1,2,3,4-tetrahydro-2(1H)-quinolinone hydrochloride hydrate,
4R-methyl-6-(2-(4-(1,2-benzisothiazol-3-yl)-piperazinyl)ethyl)-1,2,3,4-tetrahydro-2(1H)-quinolinone hydrochloride hydrate,
7-chloro-6-(2-(4-(1,2-benzisothiazol-3-yl)-piperazinyl)ethyl)-1,2,3,4-tetrahydro-2(1H)-quinolinone quarterhydrate,
6-(3-(4-(1,2-benzisothiazol-3-yl)piperazinyl)-propyl)-1,2,3,4-tetrahydro-4-methyl-2(1H)-quinolinone,
7-(2-(4-(1,2-benzisothiazol-3-yl)-piperazinyl)-ethyl)-1,3,4,5-tetrahydro-2H-1-benzazepin-2-one,
1-ethyl-6-(2-(4-(1,2-benzisothiazol-3-yl)-piperazinyl)ethyl)-1,2,3,4-tetrahydro-2(1H)-quinolinone, and
4,4-dimethyl-6-(2-(4-(1,2-benzisothiazol-3-yl)-piperazinyl)ethyl)-1,2,3,4-tetrahydro-2(1H)quinoline.

5. A pharmaceutical composition comprising a compound as claimed in any one of claims 1 to 4 or a pharmaceutically acceptable salt thereof, together with a pharmaceutically acceptable diluent or carrier.

6. A compound as claimed in any one of claims 1 to 4, or a pharmaceutically acceptable salt or pharmaceutical composition thereof, for use in medicine.

7. The use of a compound as claimed in any one of claims 1 to 4, or of a pharmaceutically-acceptable salt thereof, for the manufacture of a medicament for the treatment or prevention of psychosis or anxiety.

## Claims (Claims for the following Contracting State(s): ES, GR)

1. A process for making a compound having the formula 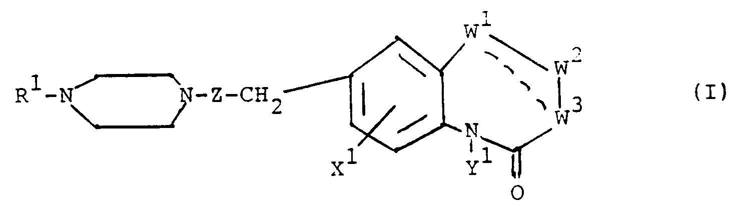 wherein W¹ is CR²R³, W² is CR⁴R⁵, W³ is CR⁶R⁷, and one of W¹, W² and W³ may be absent, and wherein the broken line extending from W¹ to W³ represents an optional bond between either W¹ and W² or W² and W³, in which case two of R², R³, R⁴, R⁵, R⁶ and R⁷ are absent; and wherein X¹ is hydrogen, halogen, (C₁-C₄)alkyl, (C₁-C₄)alkoxy, nitro, cyano, trifluoromethyl or pentafluoroethyl, or X¹ forms a heterocyclic ring with Y¹; Y¹ is hydrogen, (C₁-C₄) alkyl, phenyl or substituted phenyl, wherein said substituted phenyl is substituted with one or more substituents that are independently selected from halogen, (C₁-C₄) alkyl, (C₁-C₄) alkoxy, nitro, cyano, trifluoromethyl or pentafluoroethyl, or Y¹ forms a heterocyclic ring with X¹;
R¹ is 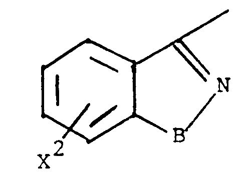 wherein B is selected from the group consisting of S, O and NY²; X² is hydrogen, halogen, (C₁-C₄) alkyl, (C₁-C₄) alkoxy, nitro, cyano, trifluoromethyl or pentafluoroethyl, or X² forms a heterocyclic ring with Y²; Y² is hydrogen (C₁-C₄) alkyl, phenyl or substituted phenyl, wherein said substituted phenyl is substituted with one or more substituents that are independently selected from halogen, (C₁-C₄) alkyl, (C₁-C₄) alkoxy, nitro, cyano, trifluoromethyl or trifluoroethyl, or Y² forms a heterocyclic ring with X²; R², R³, R⁴, R⁵, R⁶ and R⁷ are independently selected from hydrogen and lower alkyl, or any two of R², R³, R⁴ R⁵, R⁶ and R⁷ taken together with the carbon or carbons to which they are attached form a (C₃-C₇) saturated or unsaturated carbocyclic ring; and Z is (C₁-C₆)alkylene, branched (C₂-C₆)alkylene, (C₂-C₆)alkenylene or branched (C₃-C₆)alkenylene,
or a pharmaceutically acceptable salt thereof, comprising reacting a compound of the formula 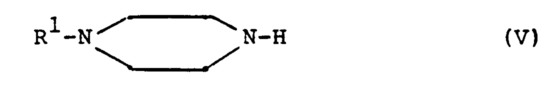 wherein R¹ is defined as above, with a compound of the formula 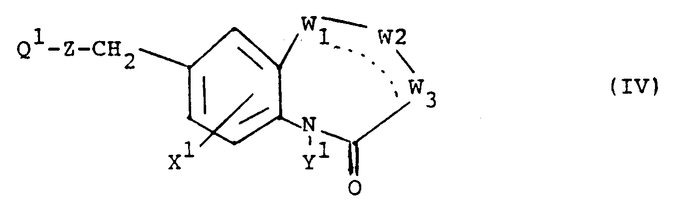 wherein W¹, W², W³, X¹, Y¹ and Z are as defined above and Q¹ is a suitable leaving group.

2. A process according to claim 1, wherein said leaving group is selected from chloro, fluoro, bromo, iodo, CH₃SO₃ and p-toluenesulfonyloxy.

3. A process according to claim 1 or claim 2, wherein the reaction is conducted in a polar solvent and in the presence of a weak base.

4. A process according to claim 3, wherein said solvent is selected from a lower alcohol, dimethylformamide, dimethylacetamide, acetonitrile and methylisobutylketone, and said weak base is selected from triethylamine and inorganic bases such as sodium carbonate and potassium carbonate.

5. A process according to any of claims 1-4, wherein the reaction is conducted at a temperature from about 0°C to about 250°C.

6. A process according to claim 5, wherein the reaction is conducted at the reflux temperature of the chosen solvent.

7. A process according to any of claims 1-6, wherein said compound of the formula IV is obtained by reducing a compound of the formula

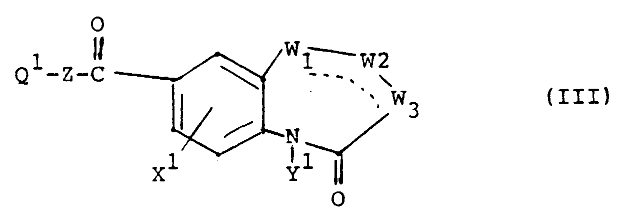

8. A process according to claim 7, wherein the reducing agent is triethylsilane in trifluoroacetic acid.

9. A process according to any of claims 7-8, wherein said compound of the formula III is obtained by reacting a compound of the formula 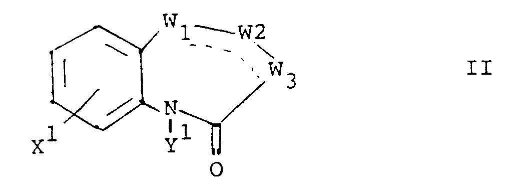 wherein W², W², W³, X¹ and Y¹ are as defined in claim 1, with a haloalkanoic acid or a haloalkanoyl halide, wherein the "halo" moieties of said haloalkanoic acid and haloalkanoyl halide are selected from fluoro, chloro, bromo and iodo, and said halide is selected from fluoride, chloride, bromide and iodide.

10. A process according to claim 9, wherein the reaction of said compound of the formula II with said haloalkanoyl halide or haloalkanoic acid is conducted using Friedel-Crafts conditions (e.g. aluminum trichloride in carbon disulfide or methylene dichloride under an inert atmosphere), or via acylation in a medium such as polyphosphoric acid, respectively.

## Patentansprüche (Patentansprüche für folgende(n) Vertragsstaat(en): AT, BE, CH, DE, DK, FR, GB, IT, LI, LU, NL, SE)

1. Verbindung der Formel 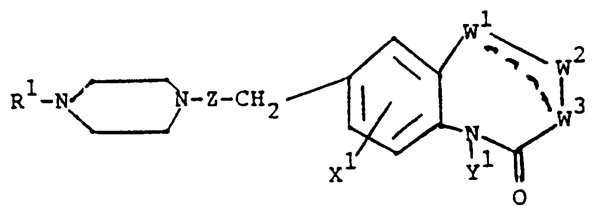 worin W¹ CR²R³ bedeutet, W² CR⁴R⁵ bedeutet, W³ CR⁶R⁷ bedeutet und einer von W¹, W² und W³ nicht vorliegen kann und worin die Strichlinie von W¹ bis W³ eine gegebenenfalls vorliegende Bindung zwischen entweder W¹ und W² oder W² und W³ darstellt, wobei in dem Fall zwei von R², R³, R⁴, R⁵, R⁶ und R⁷ nicht vorliegen und worin X¹ Wasserstoff, Halogen, (C₁-C₄)-Alkyl, (C₁-C₄)-Alkoxy, Nitro, Cyano, Trifluormethyl oder Pentafluorethyl darstellt oder X¹ einen heterocyclischen Ring mit Y¹ bildet; Y¹ Wasserstoff, (C₁-C₄)-Alkyl, Phenyl oder eine substituierte Phenylgruppe bedeutet, worin die substituierte Phenylgruppe mit einem oder mehreren Substituenten substituiert ist, die unabhängig voneinander ausgewählt sind aus Halogen, (C₁-C₄)-Alkyl, (C₁-C₄)-Alkoxy, Nitro, Cyano, Trifluormethyl oder Pentafluorethyl, oder Y¹ einen heterocyclischen Ring mit X¹ bildet;
R¹ 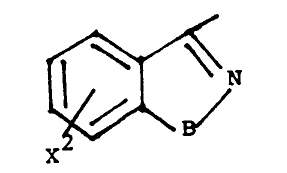 ist, worin B ausgewählt ist aus S, 0 und NY²; X² Wasserstoff, Halogen, (C₁-C₄)-Alkyl, (C₁-C₄)-Alkoxy, Nitro, Cyano, Trifluormethyl oder Pentafluorethyl bedeutet oder X² einen heterocyclischen Ring mit Y² bildet; Y² ein Wasserstoffatom, (C₁-C₄)-Alkyl, Phenyl oder eine substituierte Phenylgruppe bedeutet, worin die substituierte Phenylgruppe mit einem oder mehreren Substituenten substituiert ist, die unabhängig voneinander ausgewählt sind aus Halogen, (C₁-C₄)-Alkyl, (C₁-C₄)-Alkoxy, Nitro, Cyano, Trifluormethyl oder Trifluorethyl, oder Y² einen heterocyclischen Ring mit X² bildet; R², R³, R⁴, R⁵, R⁶ und R⁷ unabhängig voneinander ausgewählt sind aus Wasserstoff und Niederalkyl oder zwei beliebige von R², R³, R⁴, R⁵, R⁶ und R⁷ zusammengenommen mit dem Kohlenstoffatom oder Kohlenstoffatomen, an das/die sie gebunden sind, einen (C₃-C₇) gesättigten oder ungesättigten carbocyclischen Ring bilden; und Z (C₁-C₆)-Alkylen, verzweigtes (C₂-C₆)-Alkylen, (C₂-C₆)-Alkenylen oder verzweigtes (C₃-C₆)-Alkenylen bildet,
und die pharmazeutisch verträglichen Salze davon.

2. Verbindung nach Anspruch 1, mit der Maßgabe, daß wenn Y¹ Wasserstoff bedeutet und W³ nicht vorliegt, dann die heterocyclischen Ringkohlenstoffatome von W¹ und W² durch eine Kohlenstoff-Kohlenstoff-Einfachbindung verbunden sind.

3. Verbindung nach Anspruch 1, wobei Y¹ Wasserstoff bedeutet, W³ nicht vorliegt und die heterocyclischen Ringkohlenstoffatome von W¹ und W² durch eine Kohlenstoff-Kohlenstoff-Doppelbindung verbunden sind.

4. Verbindung nach Anspruch 1, wobei die Verbindung ausgewählt ist aus:
6-(2-(4-(1,2-Benzisothiazol-3-yl)piperazinyl)-ethyl)-1,2,3,4-tetrahydro-2(1H)-chinolinonhydrochloridhemihydrat,
4(R,S)-Methyl-6-(2-(4-(1,2-benzisothiazol-3-yl)-piperazinyl)ethyl)-1,2,3,4-tetrahydro-2(1H)-chinolinonhydrochloridhydrat,
4S-Methyl-6-(2-(4-(1,2-benzisothiazol-3-yl)-piperazinyl)ethyl)-1,2,3,4-tetrahydro-2(1H)-chinolinonhydrochloridhydrat,
4R-Methyl-6-(2-(4-(1,2-benzisothiazol-3-yl)-piperazinyl)ethyl)-1,2,3,4-tetrahydro-2(1H)-chinolinonhydrochloridhydrat,
7-Chlor-6-(2-(4-(1,2-benzisothiazol-3-yl)-piperazinyl)ethyl)-1,2,3,4-tetrahydro-2(1H)-chinolinonquarterhydrat,
6-(3-(4-(1,2-Benzisothiazol-3-yl)piperazinyl)propyl)-1,2,3,4-tetrahydro-4-methyl-2(1H)-chinolinon,
7-(2-(4-(1,2-Benzisothiazol-3-yl)piperazinyl)ethyl)-1,3,4,5-tetrahydro-2H-1-benzazepin-2-on,
1-Ethyl-6-(2-(4-(1,2-benzisothiazol-3-yl)piperazinyl)ethyl)-1,2,3,4-tetrahydro-2(1H)-chinolinon, und
4,4-Dimethyl-6-(2-(4-(1,2-benzisothiazol-3-yl)-piperazinyl)ethyl)-1,2,3,4-tetrahydro-2(1H)chinolin.

5. Pharmazeutische Zusammensetzung, umfassend eine Verbindung nach einem der Ansprüche 1 bis 4 oder ein pharmazeutisch verträgliches Salz davon, zusammen mit einem pharmazeutisch verträglichen Verdünnungs- oder Trägermittel.

6. Verbindung nach einem der Ansprüche 1 bis 4 oder ein pharmazeutisch verträgliches Salz davon oder eine pharmazeutische Zusammensetzung davon zur Verwendung in der Medizin.

7. Verwendung einer Verbindung nach einem der Ansprüche 1 bis 4 oder eines pharmazeutisch verträglichen Salzes davon zur Herstellung eines Arzneimittels zur Behandlung oder Verhinderung von Psychose oder Angstzustand.

## Patentansprüche (Patentansprüche für folgende(n) Vertragsstaat(en): ES, GR)

1. Verfahren zur Herstellung einer Verbindung der Formel 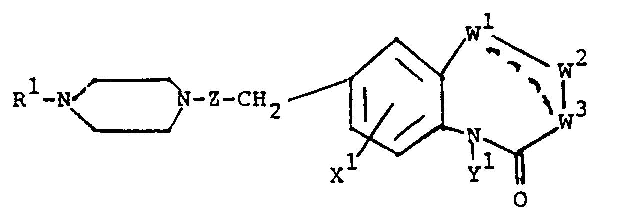 worin W¹ CR²R³ bedeutet, W² CR⁴R⁵ bedeutet, W³ CR⁶R⁷ bedeutet und einer von W¹, W² und W³ nicht vorliegen kann und worin die Strichlinie von W¹ bis W³ eine gegebenenfalls vorliegende Bindung zwischen entweder W¹ und W² oder W² und W³ darstellt, wobei in dem Fall zwei von R², R³, R⁴, R⁵, R⁶ und R⁷ nicht vorliegen und worin X¹ Wasserstoff, Halogen, (C₁-C₄)-Alkyl, (C₁-C₄)-Alkoxy, Nitro, Cyano, Trifluormethyl oder Pentafluorethyl darstellt oder X¹ einen heterocyclischen Ring mit Y¹ bildet; Y¹ Wasserstoff, (C₁-C₄)-Alkyl, Phenyl oder eine substituierte Phenylgruppe bedeutet, worin die substituierte Phenylgruppe mit einem oder mehreren Substituenten substituiert ist, die unabhängig voneinander ausgewählt sind aus Halogen, (C₁-C₄)-Alkyl, (C₁-C₄)-Alkoxy, Nitro, Cyano, Trifluormethyl oder Pentafluorethyl, oder Y¹ einen heterocyclischen Ring mit X¹ bildet;
R¹ 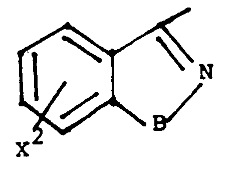 ist, worin B ausgewählt ist aus der Gruppe, bestehend aus S, 0 und NY²; X² Wasserstoff, Halogen, (C₁-C₄)-Alkyl, (C₁-C₄)-Alkoxy, Nitro, Cyano, Trifluormethyl oder Pentafluorethyl bedeutet oder X² einen heterocyclischen Ring mit Y² bildet; Y² ein Wasserstoffatom, (C₁-C₄)-Alkyl, Phenyl oder eine substituierte Phenylgruppe bedeutet, worin die substituierte Phenylgruppe mit einem oder mehreren Substituenten substituiert ist, die unabhängig voneinander ausgewählt sind aus Halogen, (C₁-C₄)-Alkyl, (C₁-C₄)-Alkoxy, Nitro, Cyano, Trifluormethyl oder Trifluorethyl, oder Y² einen heterocyclischen Ring mit X² bildet; R², R³, R⁴, R⁵, R⁶ und R⁷ unabhängig voneinander ausgewählt sind aus Wasserstoff und Niederalkyl oder zwei beliebige von R², R³, R⁴, R⁵, R⁶ und R⁷ zusammengenommen mit dem Kohlenstoffatom oder Kohlenstoffatomen, an das/die sie gebunden sind, einen (C₃-C₇) gesättigten oder ungesättigten carbocyclischen Ring bilden; und Z (C₁-C₆)-Alkylen, verzweigtes (C₂-C₆)-Alkylen, (C₂-C₆)-Alkenylen oder verzweigtes (C₃-C₆)-Alkenylen bildet,
und eines pharmazeutisch verträglichen Salzes davon, umfassend die Umsetzung einer Verbindung der Formel 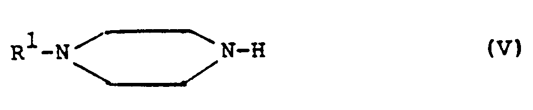 worin R¹ wie vorstehend definiert ist, mit einer Verbindung der Formel 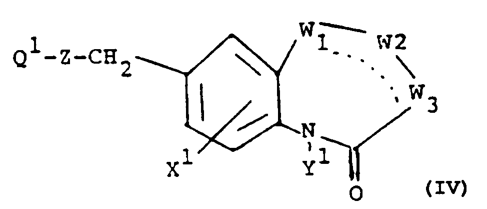 worin W¹, W², W³, X¹, Y¹ und Z wie vorstehend definiert sind und Q¹ eine geeignete Abgangsgruppe ist.

2. Verfahren nach Anspruch 1, worin die Abgangsgruppe ausgewählt ist aus Chlor, Fluor, Brom, Jod, CH₃SO₃ und p-Toluolsulfonyloxy.

3. Verfahren nach Anspruch 1 oder Anspruch 2, worin die Reaktion in einem polaren Lösungsmittel und in Gegenwart einer schwachen Base ausgeführt wird.

4. Verfahren nach Anspruch 3, wobei das Lösungsmittel ausgewählt ist aus einem niederen Alkohol, Dimethylformamid, Dimethylacetamid, Acetonitril und Methylisobutylketon und die schwache Base ausgewählt ist aus Triethylamin und anorganischen Basen, wie Natriumcarbonat und Kaliumcarbonat.

5. Verfahren nach einem der Ansprüche 1-4, wobei die Umsetzung bei einer Temperatur von etwa 0°C bis etwa 250°C ausgeführt wird.

6. Verfahren nach Anspruch 5, wobei die Umsetzung bei der Rückflußtemperatur des ausgewählten Lösungsmittels durchgeführt wird.

7. Verfahren nach einem der Ansprüche 1-6, wobei die Verbindung der Formel IV durch Reduzieren einer Verbindung der Formel 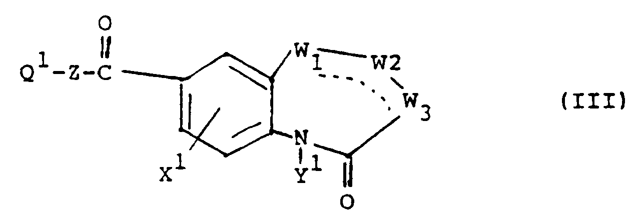 erhältlich ist.

8. Verfahren nach Anspruch 7, wobei das Reduktionsmittel Triethylsilan in Trifluoressigsäure ist.

9. Verfahren nach einem der Ansprüche 7-8, wobei die Verbindung der Formel III durch die Umsetzung einer Verbindung der Formel 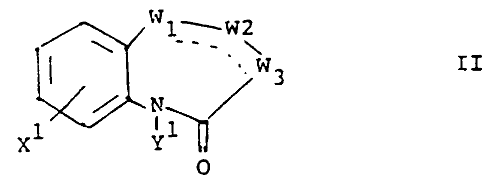 worin W¹, W², W³, X¹ und Y¹ wie in Anspruch 1 definiert sind, mit einer Halogenalkansäure oder einem Halogenalkanoylhalogenid erhältlich ist, wobei die "Halogen"reste der Halogenalkansäure und des Halogenalkanoylhalogenids ausgewählt sind aus Fluor, Chlor, Brom und Jod, und das Halogenid ausgewählt ist aus Fluorid, Chlorid, Bromid und Jodid.

10. Verfahren nach Anspruch 9, wobei die Umsetzung der Verbindung der Formel II mit dem Halogenalkanoylhalogenid oder der Halogenalkansäure unter Verwendung von Friedel-Crafts-Bedingungen (beispielsweise Aluminiumtrichlorid in Schwefelkohlenstoff oder Methylenchlorid unter einer inerten Atmosphäre) bzw. über Acylierung in einem Medium, wie Polyphosphorsäure, ausgeführt wird.

## Revendications (Revendications pour l'(les) Etat(s) contractant(s) suivant(s): AT, BE, CH, DE, DK, FR, GB, IT, LI, LU, NL, SE)

1. Composé de formule : 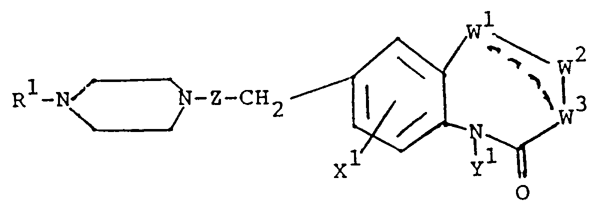 dans laquelle W¹ représente un groupe CR²R³, W² représente un groupe CR⁴R⁵, W³ représente un groupe CR⁶R⁷, et un des groupes W¹, W² et W³ peut être absent, et dans laquelle la ligne discontinue s'étendant de W¹ à W³ représente une liaison facultative entre W¹ et W² ou bien W² et W³, auquel cas deux des groupes R², R³, R⁴, R⁵, R⁶ et R⁷ sont absents ; et dans laquelle X¹ représente l'hydrogène, un halogène, un groupe alkyle en C₁ à C₄, alkoxy en C₁ à C₄, nitro, cyano, trifluorométhyle ou pentafluoréthyle, ou bien X¹ forme un noyau hétérocyclique avec Y¹ ; Y¹ représente l'hydrogène, un groupe alkyle en C₁ à C₄, phényle ou phényle substitué, ledit groupe phényle substitué portant un ou plusieurs substituants qui sont choisis indépendamment entre des groupes halogéno, alkyle en C₁ à C₄, alkoxy en C₁ à C₄, nitro, cyano, trifluorométhyle et pentafluoréthyle, ou bien Y¹ forme un noyau hétérocyclique avec X¹ ;
R¹ représente un groupe : 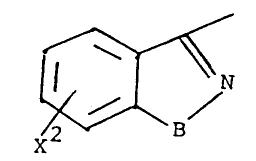 dans lequel B est choisi entre S, O et un groupe NY² ; X² représente l'hydrogène, un halogène, un groupe alkyle en C₁ à C₄, alkoxy en C₁ à C₄, nitro, cyano, trifluorométhyle ou pentafluoréthyle, ou bien X² forme un noyau hétérocyclique avec Y² ; Y² représente l'hydrogène, un groupe alkyle en C₁ à C₄, phényle ou phényle substitué, ledit groupe phényle substitué portant un ou plusieurs substituants qui sont choisis indépendamment entre des groupes halogéno, alkyle en C₁ à C₄, alkoxy en C₁ à C₄, nitro, cyano, trifluorométhyle et trifluoréthyle, ou bien Y² forme un noyau hétérocyclique avec X² ; R², R³, R⁴, R⁵, R⁶ et R⁷ sont choisis indépendamment entre l'hydrogène et un groupe alkyle inférieur, ou bien deux quelconques des groupes R², R³, R⁴, R⁵, R⁶ et R⁷ pris conjointement avec le ou les atomes de carbone auxquels ils sont fixés forment un noyau carbocyclique saturé ou insaturé en C₃ à C₇ ; et Z représente un groupe alkylène en C₁ à C₆, alkylène en C₂ à C₆ ramifié, alcénylène en C₂ à C₆ ou alcénylène en C₃ à C₆ ramifié,
et ses sels pharmaceutiquement acceptables.

2. Composé suivant la revendication 1, sous réserve que, lorsque Y¹ représente l'hydrogène et W³ est absent, les atomes de carbone du noyau hétérocyclique de W¹ et W² soient connectés par une liaison simple carbone-carbone.

3. Composé suivant la revendication 1, dans lequel Y¹ représente l'hydrogène, W³ est absent et les atomes de carbone du noyau hétérocyclique de W¹ et W² sont connectés par une double liaison carbone-carbone.

4. Composé suivant la revendication 1, qui est choisi entre :
l'hémihydrate de chlorhydrate de 6-(2-(4-(1,2-benzisothiazole-3-yl)pipérazinyl)éthyl)-1,2,3,4-tétrahydro-2(1H)-quinolinone,
l'hydrate de chlorhydrate de 4(R,S)-méthyl-6-(2-(4-(1,2-benzisothiazole-3-yl)pipérazinyl)éthyl)-1,2,3,4-tétrahydro-2(1H)-quinolinone,
l'hydrate de chlorhydrate de 4S-méthyl-6-(2-(4-(1,2-benzisothiazole-3-yl)pipérazinyl)éthyl)-1,2,3,4-tétrahydro-2(1H)- quinolinone,
l'hydrate de chlorhydrate de 4R-méthyl-6-(2-(4-(1,2-benzisothiazole-3-yl)pipérazinyl)éthyl)-1,2,3,4-tétrahydro-2(1H)-quinolinone,
le 0,25-hydrate de 7-chloro-6-(2-(4-(1,2-benzisothiazole-3-yl)pipérazinyl)éthyl)-1,2,3,4-tétrahydro-2(1H)-quinolinone,
la 6-(3-(4-(1,2-benzisothiazole-3-yl)pipérazinyl)propyl)-1,2,3,4-tétrahydro-4-méthyl-2(1H)-quinolinone,
la 7-(2-(4-(1,2-benzisothiazole-3-yl)pipérazinyl)éthyl)-1,3,4,5-tétrahydro-2H-1-benzazépine-2-one,
la 1-éthyl-6-(2-(4-(1,2-benzisothiazole-3-yl)pipérazinyl)-éthyl)-1,2,3,4-tétrahydro-2(1H)-quinolinone, et
la 4,4-diméthyl-6-(2-(4-(1,2-benzisothiazole-3-yl)pipérazinyl)éthyl)-1,2,3,4-tétrahydro-2(1H)-quinoléine.

5. Composition pharmaceutique comprenant un composé suivant l'une quelconque des revendications 1 à 4 ou un de ses sels pharmaceutiquement acceptables, en association avec un diluant ou support pharmaceutiquement acceptable.

6. Composé suivant l'une quelconque des revendications 1 à 4, ou un de ses sels pharmaceutiquement acceptables ou bien une de ses compositions pharmaceutiques, destiné à être utilisé en médecine.

7. Utilisation d'un composé suivant l'une quelconque des revendications 1 à 4, ou d'un de ses sels pharmaceutiquement acceptables, pour la production d'un médicament destiné au traitement ou à la prévention de la psychose ou de l'anxiété.

## Revendications (Revendications pour l'(les) Etat(s) contractant(s) suivant(s): ES, GR)

1. Procédé de production d'un composé de formule : 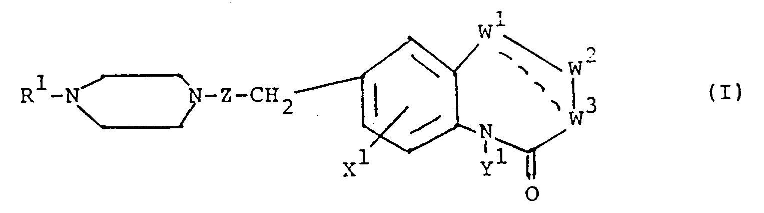 dans laquelle W¹ représente un groupe CR²R³, W² représente un groupe CR⁴R⁵, W³ représente un groupe CR⁶R⁷, et un des groupes W¹, W² et W³ peut être absent, et dans laquelle la ligne discontinue s'étendant de W¹ à W³ représente une liaison facultative entre W¹ et W² ou bien W² et W³, auquel cas deux des groupes R², R³, R⁴, R⁵, R⁶ et R⁷ sont absents ; et dans laquelle X¹ représente l'hydrogène, un halogène, un groupe alkyle en C₁ à C₄, alkoxy en C₁ à C₄, nitro, cyano, trifluorométhyle ou pentafluoréthyle, ou bien X¹ forme un noyau hétérocyclique avec Y¹ ; Y¹ représente l'hydrogène, un groupe alkyle en C₁ à C₄, phényle ou phényle substitué, ledit groupe phényle substitué portant un ou plusieurs substituants qui sont choisis indépendamment entre des groupes halogéno, alkyle en C₁ à C₄, alkoxy en C₁ à C₄, nitro, cyano, trifluorométhyle et pentafluoréthyle, ou bien Y¹ forme un noyau hétérocyclique avec X¹ ;
R¹ représente un groupe : 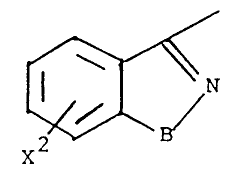 dans lequel B est choisi entre S, O et un groupe NY² ; X² représente l'hydrogène, un halogène, un groupe alkyle en C₁ à C₄, alkoxy en C₁ à C₄, nitro, cyano, trifluorométhyle ou pentafluoréthyle, ou bien X² forme un noyau hétérocyclique avec Y² ; Y² représente l'hydrogène, un groupe alkyle en C₁ à C₄, phényle ou phényle substitué, ledit groupe phényle substitué portant un ou plusieurs substituants qui sont choisis indépendamment entre des groupes halogéno, alkyle en C₁ à C₄, alkoxy en C₁ à C₄, nitro, cyano, trifluorométhyle et trifluoréthyle, ou bien Y² forme un noyau hétérocyclique avec X² ; R², R³, R⁴, R⁵, R⁶ et R⁷ sont choisis indépendamment entre l'hydrogène et un groupe alkyle inférieur, ou bien deux quelconques des groupes R², R³, R⁴, R⁵, R⁶ et R⁷ pris conjointement avec le ou les atomes de carbone auxquels ils sont fixés forment un noyau carbocyclique saturé ou insaturé en C₃ à C₇ ; et Z représente un groupe alkylène en C₁ à C₆, alkylène en C₂ à C₆ ramifié, alcénylène en C₂ à C₆ ou alcénylène en C₃ à C₆ ramifié,
ou d'un de ses sels pharmaceutiquement acceptables, comprenant la réaction d'un composé de formule : 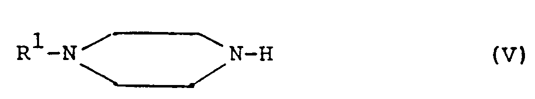 dans laquelle R¹ répond à la définition précitée, avec un composé de formule : 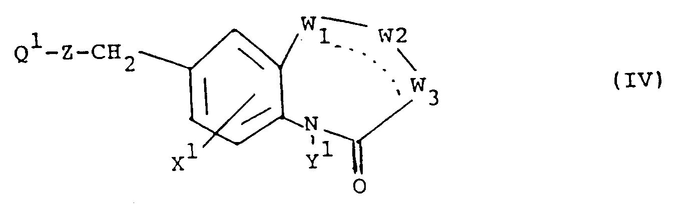 dans laquelle W¹, W², W³, X¹, Y¹ et Z répondent aux définitions précitées et Q¹ représente un groupe partant convenable.

2. Procédé suivant la revendication 1, dans lequel le groupe partant est choisi entre les groupes chloro, fluoro, bromo, iodo, CH₃SO₃ et p-toluènesulfonyloxy.

3. Procédé suivant la revendication 1 ou la revendication 2, dans lequel la réaction est conduite dans un solvant polaire et en présence d'une base faible.

4. Procédé suivant la revendication 3, dans lequel le solvant est choisi entre un alcool inférieur, le diméthylformamide, le diméthylacétamide, l'acétonitrile et la méthylisobutylcétone, et ladite base faible est choisie entre la triéthylamine et des bases inorganiques telles que le carbonate de sodium et le carbonate de potassium.

5. Procédé suivant l'une quelconque des revendications 1 à 4, dans lequel la réaction est conduite à une température d'environ 0°C à environ 250°C.

6. Procédé suivant la revendication 5, dans lequel la réaction est conduite à la température de reflux du solvant choisi.

7. Procédé suivant l'une quelconque des revendications 1 à 6, dans lequel le composé de formule IV est obtenu par réduction d'un composé de formule :

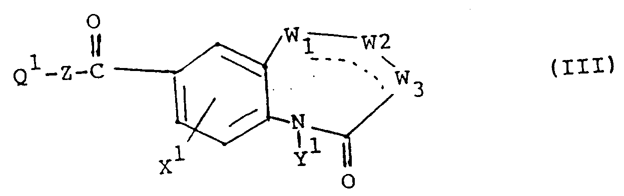

8. Procédé suivant la revendication 7, dans lequel l'agent réducteur est le triéthylsilane dans l'acide trifluoracétique.

9. Procédé suivant l'une quelconque des revendications 7 et 8, dans lequel le composé de formule III est obtenu par réaction d'un composé de formule : 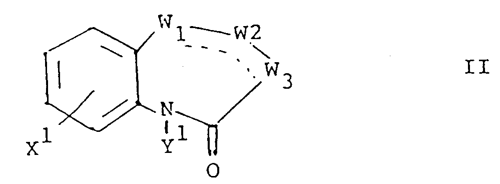 dans laquelle W¹, W², W³, X¹ et Y¹ répondent aux définitions suivant la revendication 1, avec un acide halogénalcanoïque ou un halogénure d'halogénalcanoyle, les groupements "halogéno" dudit acide halogénalcanoïque et dudit halogénure d'halogènalcanoyle étant choisis entre les groupes fluoro, chloro, bromo et iodo, et ledit halogénure étant choisi entre un fluorure, un chlorure, un bromure et un iodure.

10. Procédé suivant la revendication 9, dans lequel la réaction du composé de formule II avec l'halogénure d'halogénalcanoyle ou l'acide halogénalcanoïque est conduite respectivement dans des conditions de Friedel-Crafts (par exemple utilisation de trichlorure d'aluminium dans le disulfure de carbone ou le dichlorure de méthylène sous atmosphère inerte), ou par acylation dans un milieu tel que l'acide polyphosphorique.
